# EUROPÄISCHE PATENTSCHRIFT

(11) **EP 2 783 728 B1**
(45) Veröffentlichungstag und Bekanntmachung des Hinweises auf die Patenterteilung: **14.12.2016**
(21) Anmeldenummer: 13161014.9
(22) Anmeldetag: 26.03.2013
(51) Int. Cl.: A63B 21/00, A63B 23/18

(54) **Therapiegerät zur Behandlung von Atemwegserkrankungen**
Therapy device for the treatment of respiratory diseases
Appareil thérapeutique pour le traitement des maladies des voies aériennes

(43) Veröffentlichungstag der Anmeldung: 01.10.2014
(73) Patentinhaber: R. Cegla GmbH & Co. KG, 56410 Montabaur (DE)
(72) Erfinder: Cegla, Ulrich, 56410 Montabaur (DE)
(74) Vertreter: Engelhardt & Engelhardt

(56) Entgegenhaltungen:
- EP-A1- 0 681 853
- EP-A1- 1 987 864
- EP-B1- 2 087 927

## Beschreibung

Die Erfindung bezieht sich auf ein Therapiegerät zur Behandlung von Atemwegserkrankungen.

Beispielsweise aus der EP 2 087 927 B1 ist ein solches Therapiegerät bekannt geworden, das zur Behandlung von Atemwegserkrankungen bei Menschen eingesetzt wird. Derartige oszillierende PEP-Systeme erzeugen bei der Ausatmung aufgrund der entstehenden Druckschwankungen in den Atemwegen rhythmische Schwingungen, durch die die Atemwege erweitert und der Schleim von den Bronchialwänden abgetrennt werden.

Solche Atemwegs-Therapiegeräte bestehen üblicherweise aus einem Mundstück, das in den menschlichen Mund eingesetzt wird und durch das die Atemluft in einen an dem Mundstück fest angebrachten biegeelastischen Schlauch eingepresst ist. Das Mundstück und der Schlauch sind dabei in einem gekrümmten Rohrstück, das fest mit dem Mundstück verbunden ist, eingesetzt. Aufgrund der Biegung des Rohrstückes wird der Schlauch korrespondierend zu dem Biegeradius des Rohrstückes verformt und bereichsweise verringert sich somit der Durchtrittsquerschnitt des Schlauches, so dass in diesem Bereich ein erhöhter Luftwiderstand entsteht. Darüberhinaus wird beim Ausatmen das freie Ende des Schlauches zwischen der Innenwand des Rohrstückes hin und her bewegt, wodurch die notwendigen Druckschwankungen entstehen, denn dadurch ist gewährleistet, dass der Luftwiderstand im gekrümmten Bereich des Schlauches geringfügig verändert wird.

Solche Atemwegs-Therapiegeräte haben sich in der Praxis hervorragend bewährt und gelangen bei entsprechenden Bronchialerkrankungen erfolgreich zum Einsatz. Da jedoch die Atemwege in Richtung Lungenperipherie ständig kleiner werden und am Ende lediglich einen Durchmesser von etwa 0,5 mm aufweisen, sind niedrige Frequenzen zwischen 2 bis 6 Hz notwendig, um die Impedanz der Atemwege zu überwinden. Desweiteren sind hohe Drücke oberhalb von 10 bis 15 cmH₂O erforderlich, um die Kapillarwirkungen der kleinen Atemwege bei Entzündungen zu überwinden.

Obwohl die bekannt gewordenen Atemwegs-Therapiegeräte ein Mundstück aufweisen, dessen Position bezogen auf das Rohrstück veränderbar ist, können solche medizinischen Anforderungen mit den bekannt gewordenen Atemwegs-Therapiegeräten nicht erreicht werden.

Aus der EP 1 987 864 A1 ist ein Therapiegerät zu entnehmen, das aus einem Mundstück mit einem Durchlasskanal besteht. Dabei ist an der Öffnung des Durchlasskanals ein biegeelastischer Schlauch angebracht, der von einer Auflageplatte, die an dem Mundstück befestigt ist, abgestützt ist. An dem freien Ende der Auflageplatte ist ein Drehgelenk vorgesehen, an dem ein Widerstandskörper in unterschiedliche Winkelpositionen bezogen auf die Auflageplatte variabel einstellbar ist.

Es ist daher Aufgabe der Erfindung, ein Therapiegerät zur Behandlung von menschlichen Atemwegerkrankungen nach Anspruch 1 bereit zu stellen, mit dessen Hilfen beim Ausatmen sowohl niedrige als auch mittlere Frequenzen und Druck- bzw. Flussschwankungen in einem möglichst groß bemessenen Bereich variabel einstellbar sind.

Weitere vorteilhafte Weiterbildungen der Erfindung ergeben sich aus den Unteransprüchen.

Dadurch, dass an dem Auflagekörper eine Verstelleinrichtung angebracht ist, die relativ zu dem starren Auflagekörper bewegbar an diesem gehalten ist, und dadurch dass die Verstelleinrichtung unmittelbar oder über Zwischenglieder mit dem Schlauch in Wirkverbindung steht, kann durch eine entsprechende Veränderung der Verstelleinrichtung der Abstand des Schlauches zu dem Auflagekörper eingestellt bzw. verändert werden, wodurch sich die Krümmung des Schlauches variabel einstellen lässt. Durch eine solche Krümmungsveränderung des Schlauches verändert sich auch die Frequenz der ausgeatmeten Atemluft und der Druck den der Schlauch als Luftwiderstand der ausgeatmeten Atemluft beim Durchströmen entgegensetzt. Folglich können durch eine entsprechende Veränderung der Verstelleinrichtung die Frequenz des Schlauches sowie der Druckzustand des Schlauches an die medizinisch vorgegebenen Bedingungen angepasst werden. Obwohl diese medizinischen Bedingungen seit langen Jahren in der Fachwelt bekannt sind, hat diese keine Lösung für ein Atemwegs-Therapiegerät gefunden, durch das die medizinisch erforderlichen Frequenzen und Drücke beim Ausatmen der Atemluft derart einstellbar sind, dass in den Peripherien der Lunge eine entsprechende Schleimlösung erfolgt. Die bekannt gewordenen Atemwegs-Therapiegeräte können daher lediglich den Bronchialschleim im größeren Bereich der Lunge mit den einstellbaren Drücken und Schwingungen behandeln.

In der Zeichnung sind zwei Ausführungsvarianten eines erfindungsgemäßen Atemwegs-Therapiegeräts abgebildet, die nachfolgend näher erläutert sind. Im Einzelnen zeigt:
- Figur 1: eine erste Ausführungsvariante eines Atemwegs-Therapiegerätes bestehend aus einem Mundstück, in dem ein Durchlasskanal eingearbeitet ist, aus einem fluchtend zu dem Durchlasskanal an dem Mundstück befestigten biegeelastischen Schlauches und aus einem Auflagekörper, der eine gekrümmte Struktur aufweist und in dem beabstandet zu dem Mundstück eine Verstelleinrichtung eingesetzt ist, die mit dem Schlauch zusammenwirkt, in perspektivischer Ansicht,
- Figur 2: das Atemwegs-Therapiegerät gemäß Figur 1, in Seitenansicht,
- Figur 3: eine zweite Ausführungsvariante eines Atemwegs-Therapiegerätes bestehend aus einem Mundstück, in dem ein Durchlasskanal eingearbeitet ist, aus einem fluchtend zu dem Durchlasskanal an dem Mundstück befestigten biegeelastischen Schlauches und aus einem Auflagekörper, der eine gekrümmte Struktur aufweist und in dem beabstandet zu dem Mundstück eine Verstelleinrichtung eingesetzt ist, die mit dem Schlauch zusammenwirkt, in Seitenansicht und
- Figur 4: das Atemwegs-Therapiegerät gemäß Figur 2 mit einer zwischen dem Schlauch und der Verstelleinrichtung eingesetzten Führungsplatte.

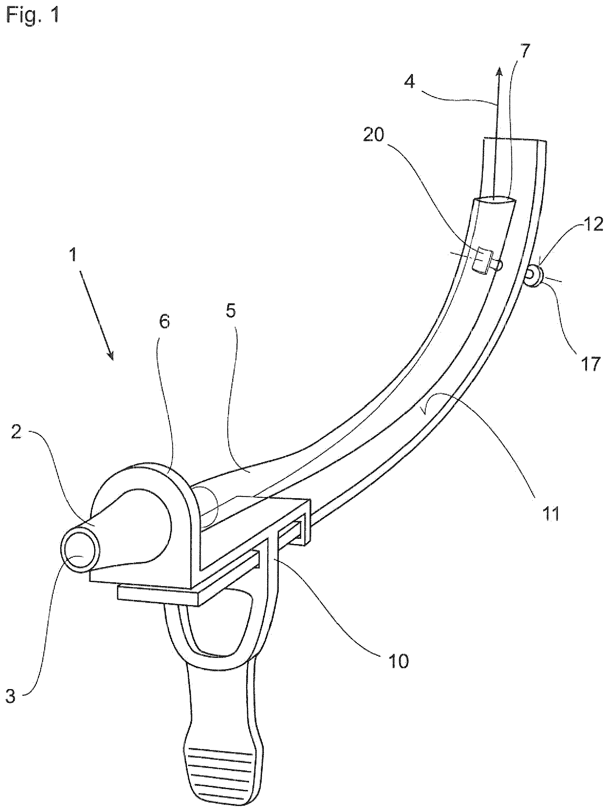

In Figur 1 ist ein Therapiegerät 1 zur Behandlung von Atemwegserkrankungen zu entnehmen, das als medizinisches Gerät zur Erzeugung von niedrig- bis mittelfrequenten Druck- und Flussschwankungen bei der Ausatmung eines Menschen eingesetzt werden kann, um die Atemwege, insbesondere die Lungenperipherie, die einen Durchmesser von etwa 0,5 mm aufweisen, zu behandeln. Das Atem-Therapiegerät 1 soll mit einer Frequenz von 2 bis 6 Hz betrieben werden, um die Impedanz der Atemwege zu überwinden, so dass hohe Drücke oberhalb von 10 bis 15 cmH₂O erzeugt werden. Dies ermöglicht eine entsprechende Kapillarwirkung in den Lungenperipherien zu überwinden, wodurch bei Entzündungen der Bronchialschleim gelöst und Bronchiolen (kleine Bronchien) erweitert werden.

Um die notwendigen Frequenzen und Drücke einstellen zu können, besteht das Therapiegerät 1 aus einem Mundstück 2, in das ein Durchlasskanal 3 eingearbeitet ist. Das Mundstück 2 wird üblicherweise zwischen der Ober- und Unterlippe eines Menschen angeordnet und durch diesen wird Atemluft, die schematisch mit Pfeilen angedeutet ist, durch den Durchlasskanal 3 des Mundstückes 2 geblasen. Die Atemluft ist mit der Bezugsziffer 4 gekennzeichnet.

Fluchtend zu der Öffnung 6 des Durchlasskanales 3 ist ein Schlauch 5 aus einem biegeelastischen Werkstoff an dem Mundstück 2 fest angeordnet, so dass die Atemluft 4 in den Schlauch 5 eingepresst ist. Der Schlauch 5 weist an dessen freien dem Durchlasskanal 3 abgewandten Ende 7 eine Durchtrittsöffnung auf, so dass die eingepresste Atemluft 4 aus dem Schlauch 5 ausströmt.

Um nunmehr zunächst den Durchtrittsquerschnitt des Schlauches 5 in einem bestimmten vorgegebenen Bereich zu krümmen, so dass dieser gegenüber dem linearen Bereich des Schlauches 5 einen geringeren Durchtrittsquerschnitt aufweist, ist ein gekrümmter oder gebogener Auflagekörper 11 vorgesehen, der fest mit dem Mundstück 2 verbunden ist. Durch die Krümmung des Auflagekörpers 11 ist der biegeelastische Schlauch 5 verformt, so dass im Bereich der Krümmung des Auflagekörpers 11 der Durchtrittsquerschnitt des Schlauches 5 gegenüber dem linearen Bereich des Schlauches 5 verringert ist. Beim Ausatmen strömt demnach die Atemluft 4 durch den Durchlasskanal 3 in den Schlauch 5 und versetzt diesen beim Ausströmen in Schwingungen. Insbesondere das freie Ende 7 des Schlauches 5 schwingt demnach hin und her und wird lediglich durch den Auflagekörper 11 begrenzt.

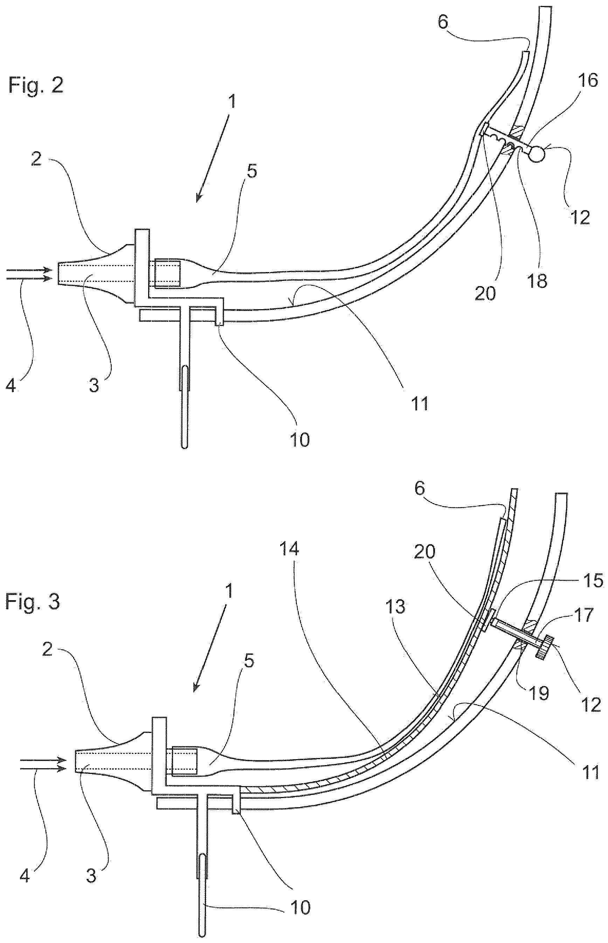

Zur Veränderung der Frequenzen und der sich einstellenden Drücke, die beim Durchströmen der Atemluft 4 durch den Schlauch 5 entstanden und von dem Patienten beim Ausatmen zu überwinden sind, ist in dem Auflagekörper 11 ferner eine relativ zu diesem bewegbare Verstelleinrichtung 12 angeordnet. Die Verstelleinrichtung 12 kann gemäß den Figuren 2 bzw. 3 entweder als Raststift 16 oder als Verstellschraube 17 ausgebildet sein. Das freie Ende des Raststiftes 16 oder der Verstellschraube 17 weist eine Auflageplatte 20 auf, die entweder unmittelbar oder über Zwischenglieder 13 mit dem Schlauch 5 in Wirkkontakt steht.

Durch die Positionsveränderung der Verstelleinrichtung 12 wird demnach der Abstand des Schlauches 5 zu dem Auflagekörper 11 variabel eingestellt, wodurch die Krümmung des Schlauches 5 unabhängig von dem Krümmungsradius des Auflagekörpers 11 einstellbar ist. In Figur 2 ist der Raststift 16 der Verstelleinrichtung 12 unmittelbar auf der Unterseite, also dem Raststift 16 zugewandten Bereich, angeordnet. Der Raststift 16 ist in in den Auflagekörper 11 eingearbeitete Rastnut 18 eingeschoben und kann entweder in Richtung des Schlauches 5 oder entgegengesetzt dazu bewegt werden, so dass die Krümmung des Schlauches 5 veränderbar ist.

In Figur 3 ist die Verstelleinrichtung 12 durch eine Verstellschraube 17 gebildet, die in eine in dem Auflagekörper 11 eingearbeiteten Innengewinde 19 eingedreht ist. Darüberhinaus ist zwischen der Auflageplatte 20 der Verstellschraube 17 und dem Schlauch 5 eine Führungsplatte 14 vorgesehen, die als Zwischenglied 13 dient. Die Führungsplatte 14 ist fest mit dem Mundstück 2 verbunden und besteht aus einem biegeelastischen und demnach verformbaren Werkstoff, so dass bei einer Positionsveränderung der Verstellschraube 17 sowohl die Führungsplatte 14 als auch der Schlauch 5 in einem weiteren oder geringerem Abstand zu dem Auflagekörper 11 positionierbar sind. Zudem begrenzt die Führungsplatte 14 den Schwingungsbereich des freien Endes 7 des Schlauches 5, denn dieser wird von der Führungsplatte 14 bereichsweise abgestützt, so dass dieser ausschließlich entgegen der von der Führungsplatte 14 gebildeten Ebene frei schwingen kann.

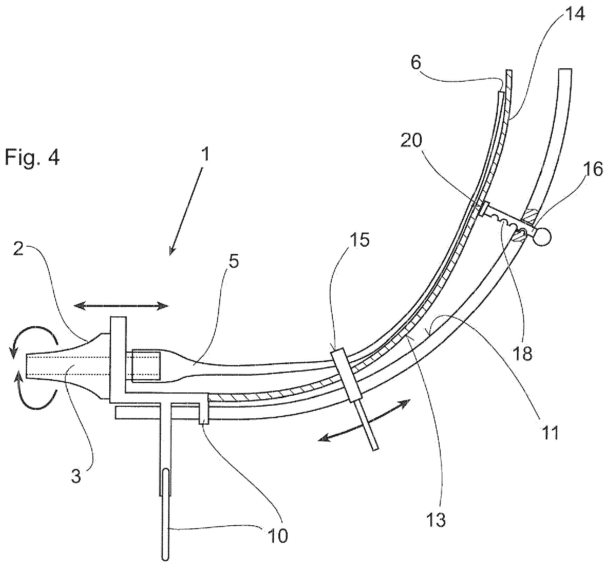

In Figur 4 ist gezeigt, dass an dem Auflagekörper 11 eine Halterung 15 vorgesehen ist, durch die die Position der Führungsplatte 14 an dem Auflagekörper 11 verschiebbar zu diesem ist. Die Halterung 15 besteht nämlich aus einem Halteteil, das verschiebbar an dem Auflagekörper 11 gelagert ist und aus einem Gelenk. Somit kann nicht nur der Abstand der Führungsplatte 14 zu dem Auflagekörper 11 durch die Verstelleinrichtung 12 variiert werden, sondern auch der sich ergebende Abstand zwischen der Verstelleinrichtung 12 und dem Drehpunkt der Führungsplatte 14, so dass dadurch der Schlauch 5 in entsprechenden Krümmungen überführt werden kann, die von dem Krümmungsradius des Auflagekörpers 11 unabhängig sind.

Um das Therapiegerät 1 an dem Mund eines Patienten manuell halten zu können, ist an dem Mundstück 2 oder an dem Auflagekörper 11 eine Halteeinrichtung 10 angeformt, die von diesem senkrecht absteht.

Die Verstelleinrichtung 12 bzw. der Raststift 16 bzw. die Verstellschraube 17 verlaufen im Wesentlichen senkrecht zu dem Auflagekörper 11; es ist jedoch vorstellbar, die Symmetrieachse der Rastnut 18 bzw. des Innengewindes 19 geneigt zu der Oberfläche des Auflagekörpers 11 in diesen einzuarbeiten.

## Patentansprüche

1. Therapiegerät (1) zur Behandlung von menschlichen Atemwegserkrankungen bestehend
- aus einem Mundstück (2), in dem ein Durchlasskanal (3) eingearbeitet ist,
- aus einem an dem Mundstück (2) befestigten Schlauch (5), der fluchtend zu der Öffnung (6) des Durchlasskanals (3) angeordnet ist und der
- aus einem biegeelastischen Werkstoff gebildet ist, dessen freies dem Durchlasskanal (3) abgewandten Ende (7) offen ausgestaltet ist und
- aus einem mit dem Mundstück (2) verbundenen gekrümmten oder gebogenen Auflagekörper (11) mit einem Krümmungsradius, wobei beabstandet zu dem Mundstück (2) in dem Auflagekörper (11) eine Verstelleinrichtung (12) vorgesehen ist, die relativ zu dem Auflagekörper (11) beweglich ist, wobei die Verstelleinrichtung (12) unmittelbar oder über Zwischenglieder (13) mit dem Schlauch (5) verbunden ist und aufgrund der Positionsveränderung der Verstelleinrichtung (12) der Abstand des Schlauches (5) zu dem Auflagekörper (11) variabel derart eingestellt ist, dass die Krümmung des Schlauches (5) unabhängig von dem Krümmungsradius des Auflagekörpers (11) einstellbar ist.

2. Therapiegerät nach Anspruch 1,
**dadurch gekennzeichnet,**
**dass** zwischen der Verstelleinrichtung (12) und dem Schlauch (5) eine als Zwischenglied (13) dienende Führungsplatte (14) angeordnet ist, dass die Verstelleinrichtung (12) auf die Führungsplatte (14) einwirkt oder trieblich mit dieser verbunden ist und dass der Schlauch (5) ganz oder zumindest bereichsweise lose auf der Führungsplatte (14) aufliegt.

3. Therapiegerät nach Anspruch 2,
**dadurch gekennzeichnet,**
**dass** die Führungsplatte (14) an dem Mundstück (2) oder an dem Auflagekörper (11) mittels einer Halterung (15) verschwenkbar befestigt ist und dass die Halterung (15) relativ zu dem Auflagekörper (11) beweglich gelagert ist.

4. Therapiegerät nach einem der vorgenannten Ansprüche,
**dadurch gekennzeichnet,**
**dass** die Verstelleinrichtung (12) als Raststift (16) oder als Verstellschraube (17) ausgestaltet ist und dass in den Auflagekörper (11) eine Rastnut (18) oder ein Innengewinde (19) eingearbeitet ist, in die die jeweilige Verstelleinrichtung (12) eingreift und trieblich mit dem Auflagekörper (11) verbunden ist.

5. Therapiegerät nach Anspruch 4,
**dadurch gekennzeichnet,**
**dass** an dem dem Schlauch (5) zugewandten freien Ende des Raststiftes (16) oder der Verstellschraube (17) eine Auflageplatte (20) angeformt ist.

6. Therapiegerät nach Anspruch 5,
**dadurch gekennzeichnet,**
**dass** die Auflageplatte (20) kreisförmig, elliptisch oder mehreckig ausgestaltet ist.

7. Therapiegerät nach einem der vorgenannten Ansprüche,
**dadurch gekennzeichnet,**
**dass** an dem Mundstück (2) oder an dem Auflagekörper (11) eine nach außen abstehende Halteeinrichtung (10) angebracht ist.

8. Therapiegerät nach einem der vorgenannten Ansprüche,
**dadurch gekennzeichnet,**
**dass** die Verstelleinrichtung (12) senkrecht oder geneigt zu dem Auflagekörper (11) ausgerichtet ist

## Claims

1. A therapeutic device (1) for the treatment of human respiratory illnesses, consisting of
- a mouthpiece (2) in which a passage duct (3) is worked,
- a hose (5) attached to the mouthpiece (2) arranged flush with the opening (6) of the passage duct (3) and which
- is formed from a flexible elastic material, the free end (7) of which facing away from the passage duct (3) is open and of
- a curved or bent support body (11) connected to the mouthpiece (2) with a curvature radius, with
an adjusting device (12) provided at a distance from the mouthpiece (2) in the support body (11), in which case the adjusting device (12) can be moved in relation to the support body (11), with
the adjusting device (12) connected to the hose (5) either directly or via intermediate elements (13) and that, and that due to the changing position of the adjusting device (12), the distance from the hose (5) to the support body (11) can be variably adjusted so that the curvature of the hose (5) can be set independently from the radius of curvature of the support body (11).

2. The therapeutic device in accordance with Claim 1,
**characterised in that,**
a guide plate (14) with varying elasticity serving as an intermediate element (13) is arranged between the adjusting device (12) and the hose (5), that the adjusting device (12) acts on the guide plate (14) or is in a driving connection with it, and that all of the hose (5) or at least an area of it lies loose on the guide plate (14).

3. The therapeutic device in accordance with Claim 2,
**characterised in that,**
the guide plate (14) on the mouthpiece (2) or on the support body (11) is in a pivoting attachment by means of a holder (15), and that the holder (15) is mounted so as to move relative to the support body (11).

4. The therapeutic device in accordance with one of the aforementioned claims,
**characterised in that,**
the adjusting device (12) is configured as a detent pin (16) or as an adjusting screw (17) and that the support body (11) has a detent groove (18) or an internal thread (19) worked into it, into which the particular adjusting device (12) engages and is in a driving connection with the support body (11).

5. The therapeutic device in accordance with Claim 4,
**characterised in that,**
a support plate (20) is formed on the free end of the detent pin (16) or the adjusting screw (17) facing the hose (5).

6. The therapeutic device in accordance Claim 5,
**characterised in that,**
the support plate (20) is circular, elliptical or polygonal.

7. The therapeutic device in accordance with one of the aforementioned claims,
**characterised in that,**
an outwardly projecting holding device (10) is attached to the mouthpiece (2) or to the support body (11).

8. The therapeutic device in accordance with one of the aforementioned claims,
**characterised in that,**
the adjusting device (12) is aligned perpendicular or at an angle to the support body (11).

## Revendications

1. Appareil thérapeutique (1) servant au traitement de maladies des voies respiratoires humaines, comprenant :
- un embout (2) dans lequel il est pratiqué un canal de passage (3),
- un tuyau flexible (5) fixé à l'embout (2), qui est aligné à l'ouverture (6) du canal de passage (3), qui est fabriqué en matériau élastique et dont l'extrémité libre (7) donnant sur le canal de passage (3), est ouverte et,
- lié à l'embout (2), un corps de support courbé (11) avec rayon de courbure, un équipement de réglage (12) étant prévu à une certaine distance de l'embout (2) dans le corps de support (11) et se laissant mouvoir relativement au corps de support (11), l'équipement de réglage (12) étant raccordé soit directement soit par l'intermédiaire d'éléments (13) au tuyau flexible (5), et où, grâce au changement de position de l'équipement de réglage (12), la distance entre le tuyau flexible (5) et le corps de support (11) est ajustée de manière variable de sorte que la courbure du tuyau flexible (5) se laisse régler indépendamment du rayon de courbure du corps de support (11).

2. Appareil thérapeutique d'après la revendication 1,
**caractérisé en ce que**,
entre l'équipement de réglage (12) et le tuyau flexible (5), il est prévu une plaque de guidage (14) servant d'élément intermédiaire (13), que l'équipement de réglage (12) agit sur la plaque de guidage (14) ou qu'il est raccordé par entraînement à celle-ci et que le tuyau flexible (5) porte entièrement ou du moins partiellement et avec du jeu sur la plaque de guidage (14).

3. Appareil thérapeutique d'après la revendication 2,
**caractérisé en ce que**
moyennant un support (15), la plaque de guidage (14) et monté de manière pivotant sur l'embout (2) ou sur le corps de support (11) et que le support (15) se laisse mouvoir relativement au corps de support (11).

4. Appareil thérapeutique d'après une des revendications précédentes,
**caractérisé en ce que**
l'équipement de réglage (12) est conçu sous la forme d'un goujon de crantage (16) ou d'une vis de réglage (17) et que dans le corps de support (11), il est pratiqué une rainure de crantage (18) ou un taraudage (19) dans lesquels s'engrène l'équipement de réglage respectif (12) lié par entraînement au corps de support (11).

5. Appareil thérapeutique d'après la revendication 4,
**caractérisé en ce que**
sur l'extrémité libre du goujon de crantage (16) ou de la vis de réglage (17), donnant sur le tuyau flexible (5), il est formé une plaque de support (20).

6. Appareil thérapeutique d'après la revendication 5,
**caractérisé en ce que**
la plaque de support (20) a une forme circulaire, elliptique ou polygonale.

7. Appareil thérapeutique d'après une des revendications précédentes,
**caractérisé en ce que**
sur l'embout (2) ou sur le corps de support (11), il est pratiqué une équipement de retient (10) saillant vers l'extérieur.

8. Appareil thérapeutique d'après une des revendications précédentes,
**caractérisé en ce que**
l'équipement de réglage (12) est orienté perpendiculairement sur ou incliné par rapport au corps de support (11).
